# EUROPEAN PATENT APPLICATION

(11) **EP 1 733 718 A1**
(43) Date of publication of application: **20.12.2006**
(21) Application number: 05447137.0
(22) Date of filing: 14.06.2005
(51) Int. Cl.: A61K 9/50

(54) **Bio-agent delivery systems based on coated degradable biocompatible hydrogels**

(71) Applicant: UNIVERSITEIT GENT, 9000 Gent (BE)
(72) Inventor: De Geest, Bruno, 9051 Sint-Denijs-Westrem (BE); De Smedt, Stefaan, 9030 Gent (BE); Demeester, Joseph, 9040 Sint-Amandsberg (BE)
(74) Representative: Bird, William Edward

(57) **Abstract**

A microcapsule of a degradable biocompatible hydrogel being coated by two or more alternating layers of at least one positively charged biocompatible polypeptide selected from the group consisting of poly-L-arginine and poly-L-ornithine and at least one biocompatible polyanion selected from the group consisting of dextran sulfate, chondroitin sulfate, poly-L-glutamic acid and poly-L-aspartic acid.

## Description

The present invention relates to the time-controlled delivery of bio-agents, e.g. biologically, medicinally, chemically, physiologically active agents such as, but not limited to, therapeutic drugs, proteins, vitamins, hormones, biocides, pesticides and the like. More precisely, the invention relates to specifically coated degradable biocompatible hydrogels for time-controlled or pulsed bio-agent release or delivery systems. In particular, the invention relates to such systems comprising a bio-agent-containing hydrogel microcapsule as a core and a polypeptide membrane surrounding said core in the form of a coating, the composition and structure of which allows for single or multiple pulse delivery of a bio-agent at specific pH conditions.

### BACKGROUND OF THE INVENTION

Currently, there is a major interest in pulsed drug delivery in which the pharmaceutical device releases the drug at a pre-defined time period. Pulsed drug release can be achieved in different ways, namely by creating a rigid, semi-permeable membrane around a core comprising the drug and a swellable component. The role of such a membrane is (i) to allow for the transport of small molecules (e.g. water molecules, ions) between the swellable component and the surrounding solution, and (ii) to prevent larger molecules (e.g. proteins, polymeric degradation products) to leave the device. With time the swelling pressure of the core gradually increases and reaches the tensile strength of the membrane, at which time the core ruptures and the drug is released. This is illustrated for instance in U.S. Patent No. 4,871,549 disclosing a so-called time-controlled explosion system in which drug release is caused by explosion of an outer membrane after a definite time period (defined as a " lag time ") which can be controlled by the kind and/or amount of swelling agent and membrane. However U.S. Patent No. 4,871,549 makes no suggestion of using a degradable hydrogel for making a time-controlled explosion system.

Other systems are also known in the art. For instance, U.S. Patent No. 6,537,584 discloses blends of chitosan, a cationic polymer, and of a second polymer, which blends once hydrated are substantially insoluble in acid or, if soluble, remain rigid in acidic conditions for a sufficient period of time to modulate drug delivery. However, U.S. Patent No. 6,537,584 makes no suggestion of a degradable biocompatible hydrogel or an outer membrane for time-controlled delivery.

U.S. Patent Application Publication No. 2004/0258753 discloses a time-controlled explosion bio-agent release system or pulsed bio-agent delivery system comprising at least (i) an outer semi-permeable membrane wherein bio-agent release or delivery is caused by explosion of said semi-permeable membrane and begins after a lag time and at least (ii) a core comprising a bio-agent and a swelling agent, wherein said swelling agent is a degradable oligomer or polymer aqueous solution or hydrogel wherein degradation occurs by cleavage of the polymer backbone and/or, in the case of a hydrogel, by cleavage of cross-linking bonds within said hydrogel. Within such a system, the lag time is at least partially controlled by the degradation rate of said degradable oligomer or polymer aqueous solution or hydrogel. U.S. Patent Application Publication No. 2004/0258753 also discloses a degradable polymer hydrogel, e.g. a degradable modified dextran, being positively or negatively charged and being further coated by means of two or more layers of one or more polyelectrolytes. Within this embodiment, the polyelectrolyte may be selected from the group consisting of pH dependent cationic polyelectrolytes, pH independent polyelectrolytes and anionic polyelectrolytes. For instance, an acrylic-modified dextran microgel was coated (examples 11-13) with a negatively charged lipid film made from stearoyloleyl phosphatidylcholine and dioleoyl trimethylammonium propane, and an acrylic-modified dextran microgel was coated (examples 14) by the consecutive adsorption of oppositely charged polyelectrolytes such as a high molecular weight chitosan or polyallylamine hydrochloride (PAH) as polycations, and sodium polystyrenesulfonate (PSS) as a polyanion.

However, further investigation of the permeability of a (PSS/PAH)-coated acrylic-modified dextran microgel to high molecular weight bio-agents such as a fluorescein isothiocyanate dextran (Mₙ from 4,000 to 20,000) showed this permeability to be pH-dependent: the coated microgels were permeable at pH 7 but impermeable at pH 9. These observations suggest that at pH 9 the polyelectrolyte membrane should be impermeable to the degradation products of the microgels, whereas at pH 7 a PSS/PAH coating is expected to be permeable to said degradation products and, consequently, a rise in osmotic pressure is not expected.

A number of synthetic polyelectrolytes have been used in electrostatic layer-by-layer self-assembly (ELBL) applications, including sodium polystyrene sulfonate (PSS), polyallylamine hydrochloride (PAH), polydiallyldimethylammonium chloride (PDDA), polyacrylamide-co-diallyldimethylammonium chloride, polyethyleneimine (PEI), polyacrylic acid (PAA), polyanetholesulfonic acid, polyvinyl sulfate (PVS), and polyvinylsulfonic acid. Such materials, however, are not generally useful for biomedical applications because they are potentially antigenic or toxic.

G. Berth et al. in Biomacromolecules (2002) 3:579-590 disclosed polyelectrolyte complexes and layer-by-layer capsules from chitosan and chitosan sulfate. B. Thierry et al. in J. Am. Chem. Soc. (2003) 125:7494-5 disclosed building on damaged arteries a nanoscale self-assembled multilayer obtained by alternating depositions of hyaluronan, a polyanion, and chitosan, a polycation, said multilayer having a thickness of about 70 nm for 5 bilayers.

U.S. Patent Application Publication No. 2005/0069950 discloses thin films comprising a plurality of layers of polypeptides having alternating charges wherein at least one of said polypeptides comprises one or more amino acid sequence motifs consisting of n amino acids, at least x of which are positively charged and none is negatively charged, or at least x of which are negatively charged and none is positively charged, wherein x is greater than or equal to approximately one-half of n. An illustrative example is an assembly of poly-L-glutamate and poly-L-lysine. However U.S. Patent Application Publication No. 2005/0069950 does not suggest using such films for coating biodegradable hydrogels.

### AIMS OF THE PRESENT INVENTION

A first problem addressed by the present invention is to provide a polyelectrolyte system for coating degradable hydrogels, in particular degradable biocompatible microgels, wherein the polyelectrolyte coating membrane is impermeable to the degradation products of said hydrogel at a pH below 7.5, i.e. a pH close to physiological conditions. The design of such a system is not easy, since it should also take into account manufacturing problems such as hydrogel particles aggregation. Indeed it was found that several biopolymers such as chitosan and poly-L-lysine (as polycations), alginic acid and hyaluronic acid (as polyanions) are unsuitable for layer-by-layer coating of hydrogel beads because they cause partial or complete aggregation of hydrogel particles. This is also confirmed by D.B.Shenoy et al. in Biomacromolecules (2003) 4:265-272 exploring combinations of chitosan or protamine sulfate (polycations) with dextran sulfate or sodium alginate or xanthan gum (polyanions) as shell components.

A second problem addressed by the present invention is to provide a polyelectrolyte system for coating degradable hydrogels, in particular degradable biocompatible microgels, wherein the coating process does not cause partial or complete aggregation of hydrogel particles.

Another problem addressed by the present invention is to provide a polyelectrolyte coating system wherein the release rate of a bio-agent entrapped within a degradable hydrogel, preferably a degradable biocompatible microgel, can be suitably tailored whatever the molecular weight or other characteristics (such as, but not limited to, water-solubility) of said bio-agent.

### SUMMARY OF THE INVENTION

The present invention is based on the unexpected finding that at least some and preferably all of the above mentioned problems can be solved simultaneously by a suitable selection of the positively charged and negatively charged alternating layers of the polyelectrolyte coating. More particularly, the present invention unexpectedly provides a degradable biocompatible hydrogel, e.g. in the form of a microcapsule, being coated by means of two or more alternating layers of at least one positively charged biocompatible polypeptide selected from the group consisting of poly-L-arginine and poly-L-ornithine and at least one biocompatible polyanion selected from the group consisting of dextran sulfate, chondroitin sulfate, poly-L-glutamic acid and poly-L-aspartic acid.

The present invention also provides microcapsules such as defined hereinabove, being filled with one or more bio-agents for use as a component of a time-controlled explosion bio-agent release system, wherein the bio-agent release or delivery begins after a pre-defined lag time. The present invention also provides different methods for making such bio-agent-loaded microcapsules. A first method comprises loading a suitable degradable biocompatible hydrogel with a suitable amount of said one or more bio-agents, e.g. in the form of a microcapsule, and then coating said bio-agent-loaded microcapsule according to the ELBL technique by alternatively depositing layers of at least one positively charged biocompatible polypeptide selected from the group consisting of poly-L-arginine and poly-L-ornithine and at least one biocompatible polyanion selected from the group consisting of chondroitin sulfate, poly-L-glutamic acid and poly-L-aspartic acid, thus forming a self-exploding microcapsule. A second method comprises a first step of making a core of a suitable degradable biocompatible hydrogel, e.g. in the form of a microcapsule, a second step of coating said microcapsule according to the ELBL technique by alternatively depositing layers of poly-L-arginine and layers of dextran sulfate, a third step of degrading a substantial portion of the hydrogel core thus forming a hollow microcapsule, and finally a fourth step of loading the hollow microcapsule with one or more bio-agents.

Finally, the present invention provides a time-controlled explosion bio-agent release system or pulsed bio-agent delivery system comprising at least (i) an outer semi-permeable membrane wherein the bio-agent release or delivery is caused by disruption or explosion of the said membrane and begins after a lag time and at least (ii) a core comprising a bio-agent and a swelling agent responsible for the disruption or explosion of said semi-permeable membrane, said swelling agent being a degradable oligomer or polymer in the form of an aqueous solution or a hydrogel wherein degradation occurs by cleavage of the polymer backbone and/or, in the case of a hydrogel, by cleavage of cross-linking bonds within the said hydrogel, characterised in that said outer semi-permeable membrane (i) consists of two or more alternating layers of at least one positively charged biocompatible polypeptide selected from the group consisting of poly-L-arginine and poly-L-ornithine and at least one biocompatible polyanion selected from the group consisting of chondroitin sulfate, poly-L-glutamic acid and poly-L-aspartic acid.

### DETAILED DESCRIPTION OF THE INVENTION

The present invention provides time-controlled delivery of bio-agents, e.g. biologically, medicinally, chemically, physiologically active agents such as, but not limited to, therapeutic drugs, proteins, vitamins, hormones, biocides, pesticides and the like. More precisely, the invention provides specifically coated degradable biocompatible hydrogels for time-controlled or pulsed bio-agent release or delivery systems. In particular, the invention provides such systems comprising a bio-agent-containing hydrogel microcapsule as a core and a polypeptide membrane surrounding said core in the form of a coating, the composition and structure of which allows for single or multiple pulse delivery of a bio-agent at specific pH conditions.

For example, the present invention provides the use of selected polyelectrolyte complexes as a coating for a degradable hydrogel capsule, preferably a degradable biocompatible microcapsule, for making a time-controlled explosion bio-agent release system or a pulsed bio-agent delivery system comprising at least one biologically active agent, wherein bio-agent release or delivery begins after a predetermined time (the so-called " lag time ") and occurs at a pH below 7.5. Depending upon the construction of the release or delivery system, i.e. in particular depending upon selected parameters such as the degradable hydrogel oligomer or polymer component, the polyelectrolyte coating components, the particular bio-agent(s), the bio-agent (e.g. drug) loading ratio and the intended route of administration, the lag time may vary within broad ranges from about one hour to two weeks, preferably from about 1 to 24 hours. The present invention also provides the use of selected polyelectrolyte complexes for making hollow microcapsules that may be filled with one or more bio-agents.

*In situ,* e.g. *in vivo,* degradation of a degradable oligomer or polymer aqueous solution or hydrogel may occur by cleavage (i.e. usually hydrolysis) of the polymer backbone or by cleavage (i.e. usually hydrolysis) of cross-linking bonds (usually covalent bonds) within the hydrogel, depending on the chemical type of the said oligomer or polymer. Whatever the degradation mechanism may be, the lag time, i.e. the time period after which the coating ruptures, is at least partially controlled by the degradation rate of the said degradable oligomer or polymer aqueous solution or hydrogel. The *in situ,* e.g. *in vivo,* degradable oligomer or polymer hydrogel for use in this invention may be any linear or branched water-soluble polymer backbone having at least two termini, at least one of the said termini being optionally covalently bonded to a linker, wherein at least one of the polymer backbone and the linker comprise a hydrolytically or enzymatically degradable linkage. The term " linkage " or "linker" is used herein to refer to groups or bonds that normally are formed as the result of a chemical reaction and typically are covalent linkages. Hydrolytically degradable linkages means that the linkages are degradable in water or in aqueous solutions at useful pHs, e.g. under physiological conditions, including for example in blood. Enzymatically degradable linkages means that the linkage can be degraded by one or more enzymes. For instance, the *in vivo* degradable oligomer or polymer hydrogel for use in this invention may be a polypeptide, provided that said polypeptide be *in vivo* degradable by an enzyme, such as a protease, which is present in the part of the body where membrane disruption is desired.

In a specific embodiment of this invention, the degradable biocompatible oligomer or polymer hydrogel may be selected from the group consisting of disaccharides (such as for instance sucrose), oligosaccharides (such as for instance p-nitrophenyl-penta-N-acetyl-chitopentaoside) and polysaccharides, all of them being enzymatically cleavable. Such di-, oligo- and polysaccharides may have any molecular weight ranging from about 100 to about 1,000,000, preferably from about 200 to about 200,000, and more preferably from about 1,000 to 40,000. Although the invention is not limited to such a kind of *in vivo* degradable biocompatible polymer hydrogels, the present invention will now be explained in more detail by reference to degradable modified dextran hydrogels which are enzymatically cleavable by dextranase. As is well known, dextran is a high molecular weight (about 15,000 to 150,000) polysaccharide containing α-glucopyranose units which may be produced from the action of *Leuconostoc mesenteroides* onto saccharose. Dextran may be chemically modified by reaction with functional α-β ethylenically unsaturated acid esters such as functional acrylates and methacrylates. For instance, methacrylated dextran (hereinafter referred to as dex-MA) may be obtained by coupling glycidyl methacrylate to dextran, as disclosed by Van Dijk et al. in Macromolecules (1995) 28:6317-6322. Dextran may also be modified by one or more (C₁₋₈ alkyl) acrylate or methacrylate by reacting dextran with an epoxy (meth)acrylate such as the superior homologues of glycidyl acrylate or methacrylate. Within the polymer aqueous solutions or hydrogels of such (meth)acrylated dextrans, degradation occurs by cleavage of the polymer backbone, more specifically by the enzymatic action of dextranase and/or by hydrolysis of the carbonate ester link formed between the methacrylate group and the dextran molecule.

Dextran may also be modified by means of at least one (hydroxy-C₁₋₈ alkyl) acrylate or methacrylate, such as for instance hydroxyethyl methacrylate, thus leading to a structure which may be represented by the following formula:

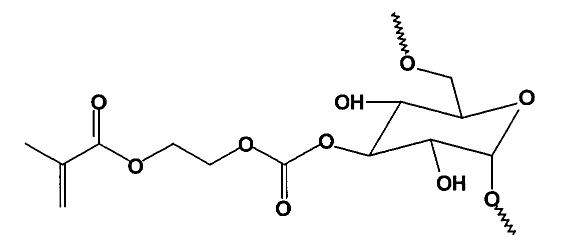

Dextran may also be modified by means of other α,β-ethylenically unsaturated entities, such as for instance acrylamides and methacrylamides, provided that that the bonds thus formed are degradable.

In polymer solutions and hydrogels from the latter modified dextran, the formation of covalent cross-linking bonds is a common feature and, hence, degradation occurs mainly by cleavage (hydrolysis) of the said cross-linking bonds within the hydrogel.

Preferably the degree of substitution (i.e. the number of chemically modifying groups, e.g. (meth)acrylic groups, per 100 glucopyranose residues of dextran) of the modified dextran used as a hydrogel core in the present invention is between about 2 and 10.

After the chemical modification of dextran, the resulting modified dextran may be dissolved in a buffer at a suitable pH, e.g. usually a pH between about 6.5 and 8.5, and the resulting aqueous solution may then be radically polymerized in the presence of a suitable soluble catalyst or catalytic system comprising, for example, N,N,N',N'-tetramethylene-ethylenediamine (hereinafter TEMED) and potassium persulfate (hereinafter KPS) until a hydrogel is obtained. Gelation can also be obtained by photopolymerisation in the absence or in the presence of a photo-initiator. Catalysts and photo-initiators suitable for this purpose are well known in the art.

Other examples of degradable oligomers or polymers that are suitable for use as hydrogel cores in the present invention include, but are not limited to, cyclodextrins and modified cyclodextrins that are enzymatically cleavable by amylase. Cyclodextrins and modified cyclodextrins, in particular their pharmaceutical grades, are well known in the art and are available from a variety of commercial sources. They may be collectively referred as starch cyclic degradation products containing 6 to 8 glucose residues, or alternatively as cyclic oligosaccharides composed of L-glucose molecules linked by α or β osidic bonds having a toric form. A suitable representative embodiment of modified cyclodextrins consists of hydroxypropyl-β-cyclodextrin.

Other examples of degradable biocompatible oligomer or polymer hydrogels that are hydrolytically degradable and thus suitable as a core for carrying out the present invention include, but are not limited to, hydrogels based on synthetic polymer backbones from substantially non-immunogenic polymers, such as polyether polyols, including those with two or more hydroxyl groups derived from polyethylene glycol (PEG) or a copolymer of ethylene oxide and an alkylene oxide (e.g. propylene oxide) with a degree of polymerization up to about 500. For instance the sequence present in the said polyether polyol may be represented by the formula - O - R ― O - , wherein R may be an alkylene group possibly substituted with one or more hydroxy groups or alternatively R may be 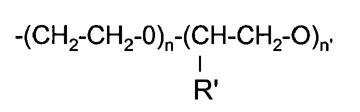 wherein R' is alkyl group with up to 4 carbon atoms, preferably methyl, n is an integer up to about 200, and n' is an integer up to about 100. However, it should be understood that other related polymers are also suitable for use as a degradable biocompatible hydrogel core in the practice of the present invention. PEG is typically clear, colorless, odorless, soluble in water, stable to heat, inert to many chemical agents, does not hydrolyze or deteriorate, and is generally nontoxic. Poly(ethylene glycol) is considered to be biocompatible, i.e. capable of coexistence with living tissues or organisms without causing harm. More specifically, PEG is non-immunogenic, i.e. PEG does not tend to produce an immune response in the human or animal body. When attached to a molecule having some desirable function in the human or animal body, such as a biologically active agent, PEG tends to mask said bio-agent and can reduce or eliminate any immune response so that the organism can tolerate the presence of said bio-agent. PEG conjugates tend not to produce a substantial immune response or cause clotting or other undesirable effects. PEG, preferably PEG having a molecular weight of from about 200 to about 100,000, may thus suitably be used as the polymer backbone of the hydrogel core and should therefore also be regarded as a useful degradable polymer in the practice of the present invention.

The polymer backbone of the hydrogel core can be linear or branched. Branched polymer backbones are generally known in the art. Typically, a branched polymer has a central branch core moiety and a plurality of linear polymer chains linked to the central branch core. PEG is commonly used in branched forms that can be prepared by addition of ethylene oxide to various polyols, such as glycerol, pentaerythritol and sorbitol. The central branch moiety can also be derived from several amino acids, such as lysine. The branched polyethylene glycols can be represented in general form as R(--PEG--OH)ₘ in which R represents the core moiety, such as glycerol, pentaerythritol or sorbitol, and m is an integer which represents the number of branches. Other suitable embodiments include PEG coupled to polylactic acid or polyglycolic acid in the form of diblock copolymers or triblock copolymers.

Many other polymers are also suitable for building the degradable biocompatible hydrogels used as a core for building the delivery system, e.g. the microcapsules, of the present invention. These polymers can be either in linear form or branched form, and include in their structure, but are not limited to, other poly(alkylene glycol), such as poly(propylene glycol), copolymers of ethylene glycol and propylene glycol and the like, poly(oxyethylated polyol), poly(olefinic alcohol), poly(vinylpyrrolidone), poly (hydroxypropylmethacrylamide), poly(α-hydroxy acid), poly(vinyl alcohol), poly-phosphazenes, polyoxazolines; polymers and copolymers (whether random, block, segmented or grafted) of lactones such as ε-caprolactone, glycolide, L-lactide, D-lactide, meso-lactide, 1,4-dioxan-2-one, trimethylene carbonate (1,3-dioxan-2-one), χ-butyrolactone, δ-valerolactone, 1,5-dioxepan-2-one, 1,4-dioxepan-2-one, 3-methyl-1,4-dioxan-2,5-dione, 3,3 diethyl-1,4-dioxan-2,5-one, ε-decalactone, pivalolactone and 4,4-dimethyl -1,3-dioxan-2-one and the like ; several embodiments of such copolymers have been described by, among others, U.S. Patent No. 5,951,997, U.S. Patent No. 5,854,383 and U.S. Patent No. 5,703,200 and shall be considered as being within the scope of the present invention ; hydroxy-terminated polyorthoesters obtainable for instance by the addition reaction of a diol (e.g. an alkylenediol such as ethylenediol, trimethyleneglycol, tetramethyleneglycol, pentamethyleneglycol, hexanediol-1,6 and the like, or a cycloalkyldiol such as 1,4-cyclohexanedimethanol or 1,4-cyclohexanediol) or polyethyleneglycol onto a diketene acetal ; such a method for a hydroxy-terminated polyorthoester is well known in the art and is described, starting from 3,9-bis(ethylidene-2,4,8,10-tetraoxaspiro[5,5] undecane, by J.Heller et al. in Macromolecular Synthesis 11 : 23-25 ; Hydroxy-terminated polyacetals obtainable for instance by the condensation reaction of at least a diol (such as hereinabove mentioned) and a divinylether as is well known in the art ; for instance, U.S. Patent No. 4,713,441 describes unsaturated, linear, water-soluble polyacetals having molecular weights from about 5,000 to about 30,000 which may be formed by condensing a divinylether, a water-soluble polyglycol and a diol having a (preferably pendant) unsaturation, which may be further converted to hydrogels, for instance by using a free-radical initiator in order to copolymerize the double bonds in the polyacetal with a monomeric compound having a reactive double bond. Another typical procedure for this kind of polyacetals may be found in Heller et al., Journal of Polym.Science, Polym.Letters Edition (1980) 18 :293-7, starting from 1,4-divinyloxybutane or diethyleneglycol divinylether. French patent No. 2,336,936 further refers to crosslinked polyacetals formed by condensing diols or polyols with 3,4-dihydro-2H-pyran-2-ylmethyl-3,4-dihydro-2H-pyran-2-ylcarboxylate which may also be used in the present invention.

Other examples of degradable oligomer or polymer hydrogels being hydrolytically degradable and thus suitable for carrying out the present invention also include macromers based on the above mentioned synthetic polymer backbones and further including one or more polymerizable region(s) containing for instance polymerizable end groups such as ethylenic and/or acetylenic unsaturations. The choice of said polymerizable end groups will be dictated by the need for rapid polymerization and gelation. Therefore, namely because they can easily be polymerized while using various polymerization initiating systems, well known in the art, vinyl groups such as, but not limited to, acrylate, methacrylate, acrylamide and methacrylamide groups are preferred for performing the macromer embodiment of this invention.

The molecular weight of the degradable hydrogel polymer backbone is typically in a range from about 100 to about 100,000, preferably from about 6,000 to about 80,000.

The skilled person understands that the foregoing list of non-immunogenic polymer backbones is by no means exhaustive but merely illustrative, and that all polymeric materials having the qualities described above are also contemplated within the scope of the present invention.

In one embodiment, the degradable polymer solution or hydrogel is then used in the form of a coated microcapsule, i.e. a capsule having a size from about 1 µm to about 100 µm, as a component of ― or as an intermediate for making - a time-controlled explosion bio-agent release system or a pulsed bio-agent delivery system, the latter further comprising at least one biologically active agent and a specific biocompatible coating, wherein said biocompatible coating is designed to allow self-explosion at a pH below 7.5, in particular at physiological conditions. Said coating may advantageously be constructed as a semi-permeable membrane, i.e. a membrane which is permeable to ions and water, but impermeable to the one or more bio-agents and the biocompatible hydrogel degradation products.

An important feature of this embodiment of the present invention is a hydrogel coating consisting of two or more alternating layers of at least one positively charged biocompatible polypeptide selected from the group consisting of poly-L-arginine and poly-L-ornithine and at least one biocompatible polyanion selected from the group consisting of chondroitin sulfate, poly-L-glutamic acid and poly-L-aspartic acid. Preferably, the number of such alternating layers is from about 3 to about 10, more preferably from about 4 to about 6. Selecting the number of alternating layers above about 10 may result in a coating that is too strong to rupture, at least within a reasonable or useful period of time. Alternatively, a number of alternating layers that is too low may result in premature leaking of the bio-agent or in bio-agent release through diffusion rather than through explosion.

Preferably, the molecular weight of the biocompatible polyanion used in the constitution of the alternating layers is within a range from about 5,000 to about 100,000, more preferably from about 15,000 to about 50,000. Also preferably, the molecular weight of the positively charged biocompatible polypeptide used in the constitution of the alternating layers is within a range from about 50,000 to about 200,000, more preferably from about 70,000 to about 150,000.

For the construction of self-exploding capsules, it is preferred to use a degradable hydrogel coating consisting of two or more alternating layers of at least one positively charged biocompatible polypeptide selected from the group consisting of poly-L-arginine and poly-L-ornithine and at least one biocompatible polyanion selected from the group consisting of chondroitin sulfate, poly-L-glutamic acid with a molecular weight below 50,000 and poly-L-aspartic acid. For the construction of hollow capsules in another embodiment of the present invention, it is preferred to use a hydrogel coating consisting of two or more alternating layers of dextran sulfate and poly-L-arginine. By using a hydrogel coating consisting of two or more alternating layers of poly-L-glutamic acid with a molecular weight above 50,000 and poly-L-arginine, it is also possible to obtain a blend of self-exploding capsules and hollow capsules, although such a blend has limited utility in practice because it is insufficiently well-defined.

In a more preferred embodiment of the present invention, the positively charged biocompatible polypeptide used in the constitution of the alternating layers of the coating consists of poly-L-arginine, being a strong polyelectrolyte with no pH-dependent behaviour.

Providing the specific polyelectrolyte coating of the present invention onto a degradable biocompatible hydrogel may be achieved by any suitable means including, but not limited to, the well known Layer-by-Layer Electrostatic Self Assembly (ELBL) procedure. The success of this manufacturing procedure may be conveniently checked by any quality control means, including using confocal laser scanning microscopy (CSLM) and measuring the electrophoretic mobility of the microcapsules before and after electrostatic coating. The number of adsorption steps in this multi-step procedure is not particularly limited but, consistently with the desirable number of alternating layers (such as mentioned above), is preferably from 6 to about 20, more preferably from 8 to 12. When the degradable biocompatible hydrogel core is positively charged, the electrostatic adsorption procedure is started from a biocompatible polyanion. When the degradable biocompatible hydrogel core is negatively charged, the electrostatic adsorption procedure is started from a positively charged biocompatible polypeptide.

According to the second embodiment of this invention, hydrolysis or degradation of the polyelectrolyte-coated biocompatible hydrogel may be achieved or accelerated, specifically for making hollow microcapsules, by bringing said polyelectrolyte-coated hydrogel in contact with a suitable amount of an alkaline medium. For instance it was observed that, after dipping a polyelectrolyte-coated hydrogel of this invention into a 0.5 M NaOH solution, maximum swelling of the gel occurs within about 1 minute and, due to the increase in osmotic pressure caused by core degradation, stretching of the particle surface reduces the coating thickness and thus increases permeability.

In the preferred embodiments of the invention, the degradable polymer biocompatible hydrogel used as a core may have an average size within a range from about 2 µm to about 10 µm, and/or a size distribution with a dispersity from about 1.1 to about 3.0, preferably from 1.2 to 2.0. The multi-layer coating serving as an outer shell or semi-permeable membrane (being useful, among other applications, in a pulsed drug delivery system or time-controlled explosion drug release system) may suitably have a thickness within a range from about 10 nm to about 100 nm, preferably from 20 to 50 nm, as determined by standard analytical or imaging techniques well known in the art.

In view of the main use of the present invention, the degradable biocompatible hydrogel constituting the core of the microcapsule is preferably loaded with one or more bio-agents. The term " bio-agent " as used herein, unless specified otherwise, is intended to mean any substance having biological activity in animals (preferably human beings) or in plants such as, but not limited to, substances selected from the group consisting of therapeutic and prophylactic drugs and synthetic molecules, proteins, nucleic acids, vitamins, hormones, nutrients, aromas (fragances), fertilisers, anti-microbial agents, insecticides, fungicides, herbicides and pesticides, especially these where pulsed delivery is desirable for improving efficiency of the biological activity involved.

The bio-agent loading ratio of the microcapsule, i.e. the weight ratio between the one or more bio-agents and the microcapsule core, is an important feature of the present invention. Preferably the weight proportion of said one or more bio-agents within the degradable biocompatible hydrogel of a self-exploding microcapsule is from about 0.5 % to about 10 %, more preferably from about 1 % to about 8 %, most preferably from about 1.5 % to about 5 % by weight. Bio-agent loading ratios below 0.5 % by weight of the degradable biocompatible hydrogel of a self-exploding microcapsule can also easily be achieved but are of lesser importance for commercially practical reasons. Bio-agent loading ratios above 10 % by weight of the degradable biocompatible hydrogel of a self-exploding microcapsule are more difficult to achieve without encountering the problem of bio-agent release by diffusion significantly competing with the desired bio-agent release by sudden rupture of the coating and subsequent explosion.

When the coated microcapsule of this invention is a hollow capsule obtained after substantial degradation of the degradable biocompatible hydrogel core, it may afterwards be filled with a bio-agent-containing composition, e.g. a pharmaceutical or veterinary composition, comprising one or more bio-agents in admixture with one or more pharmaceutically acceptable excipients such as detailed hereinafter. In this situation, the bio-agent loading ratio of the microcapsule may be significantly higher than in the case of a self-exploding microcapsule, i.e. it may be as high as 50 % by weight, depending upon the relevant bio-agent and the type of pharmaceutically acceptable excipients usually required for its formulation. Methods for filling hollow capsules are already known in the art and may successfully be used in this invention, e.g.:
- the method based on a change in solvent polarity, for instance using a 1:1 mixture of water and ethanol such as described by Y.M. Lvov in *Nanoletters* (2001), and
- a method using co-precipitation and porous calcium carbonate as a sacrificial template such as described by D.V. Volodkin in Biomacromolecules (2004) or by A.I. Petrov in Biotechnology Progress (2005).

As evidenced by the following examples, the present invention proves to be successful with bio-agents having molecular weights in a very broad range, including rather small synthetic molecules or oligopeptides (including from about 3 to about 8 amino-acid residues) with molecular weights from about 300 to about 800, but also large molecules including polypeptides with molecular weights up to about 2,000,000, more preferably up to about 200,000.

The therapeutic bio-agent used in the present invention may be selected for its specific properties such as for instance its anti-thrombotic, anti-inflammatory, anti-proliferative, anti-viral (e.g. anti-retroviral), anti-histaminic or anti-microbial efficiency. The latter include for instance, but are not limited to, anti-microbial agents such as broad spectrum antibiotics for combating clinical and sub-clinical infection, for example gentamycin, vancomycine and the like. Other suitable therapeutic agents are naturally occurring or synthetic organic or inorganic compounds well known in the art, including non-steroidal anti-inflammatory drugs, proteins and peptides (produced either by isolation from natural sources or recombinantly), hormones (for example androgenic, estrogenic and progestational hormones such as oestradiol, and gonadotropin releasing hormone for inducing fertility), bone repair promoters, carbohydrates, antineoplastic agents, antiangiogenic agents, vaso-active agents, anticoagulants, immunomodulators, cytotoxic agents, antiviral agents, antibodies, neurotransmitters, oligonucleotides, lipids, plasmids, DNA and the like. Suitable therapeutically active proteins include e.g. fibroblast growth factors, epidermal growth factors, platelet-derived growth factors, macrophage-derived growth factors such as granulocyte macrophage colony stimulating factors, ciliary neurotrophic factors, tissue plasminogen activator, B cell stimulating factors, cartilage induction factor, differentiating factors, growth hormone releasing factors, human growth hormone, hepatocyte growth factors, immunoglobulins, insulin-like growth factors, interleukins, cytokines, interferons, tumor necrosis factors, nerve growth factors, endothelial growth factors, osteogenic factor extract, T cell growth factors, tumor growth inhibitors, enzymes and the like, as well as fragments thereof. Suitable diagnostic agents include conventional imaging agents (for instance as used in tomography, fluoroscopy, magnetic resonance imaging and the like) such as chelates of a transition metal (e.g. a radioactive metal selected from the group consisting of ^{99m}Tc, ¹¹¹In, ⁶⁷Ga, ⁹⁰Y, ¹⁸⁶Re and ¹⁸⁸Re or a non-radioactive metal selected from gadolinium, manganese and iron).

Suitable anti-microbial agents include, but are not limited to, halogenated phenols, chlorinated diphenylethers, aldehydes, alcohols such as phenoxyethanol, carboxylic acids and their derivatives, organometallic compounds such as tributyltin compounds, iodine compounds, mono- and polyamines, sulfonium and phosphonium compounds; mercapto compounds as well as their alkaline, alkaline-earth and heavy metal salts; ureas such as trihalocarbanilide, isothia- and benzisothiazolone derivatives.

Suitable insecticides include, but are not limited to, natural ones, e.g. nicotine, rotenone, pyrethrum and the like, as well as synthetic ones like chlorinated hydrocarbons, organophosphorus compounds, biological insecticides (e.g. products derived from *Bacillus thuringiensis),* synthetic pyrethroids, organosilicon compounds, nitro-imines and nitromethylenes.

Suitable fungicides include, but are not limited to, dithiocarbamates, nitrophenol derivatives, heterocyclic compounds (such as thiophthalimides, imidazoles, triazines, thiadiazoles, triazoles and the like), acylalanines, phenylbenzamides, and tin compounds.

Suitable herbicides include, but are not limited to, trichloroacetic and aromatic carboxylic acids and their salts, substituted ureas and triazines, diphenyl ether derivatives, anilides, uraciles, nitriles and the like.

Suitable fertilisers include, but are not limited to, ammonium sulfate, ammonium nitrate, ammonium phosphate and the like, and mixtures thereof.

Therapeutic agents which are advantageously delivered according to the present invention belong to all permeability and solubility classes of the Biopharmaceutical Classification System according to G. Amidon et al. in Pharm. Res. (1995) 12:413-420. As will be appreciated by those skilled in the art, these drugs belong to various therapeutic classes including, but are not limited to, β-blockers, calcium antagonists, ACE inhibitors, sympathomimetic agents, hypoglycaemic agents, contraceptive agents, α-blockers, diuretic agents, antihypertensive agents, anti-psoriasis agents, bronchodilator agents, corticosteroids, anti-mycotic agents, salicylates, cytostatic agents, antibiotic agents, antiviral agents, antihistamines, UV-absorbers, chemo-therapeutic agents, antiseptic agents, estrogens, scar treatment agents, antifungal agents, antibacterial agents, antifolate agents, cardiovascular agents, nutritional agents, antispasmodic agents, analgesics and the like.

The present invention is suitable e.g. for the time-controlled delivery of one or more of the following therapeutic or cosmetic agents: acebutolol, acetylcysteine, acetylsalicylic acid, acyclovir, alfuzosine, alprazolam, alfacalcidol, allantoin, allopurinol, alverine, ambroxol, amikacin, amiloride, aminoacetic acid, amiodarone, amitriptyline, amlodipine, amoxicillin, ampicillin, ascorbic acid, aspartame, astemizole, atenolol, beclomethasone, benserazide, benzalkonium hydrochloride, benzocaine, benzoic acid, betamethasone, bezafibrate, biotin, biperiden, bisoprolol, bromazepam, bromhexine, bromocriptine, budesonide, bufexamac, buflomedil, buspirone, caffeine, camphor, captopril, carbamazepine, carbidopa, carboplatin, cefachlor, cefalexin, cefatroxil, cefazolin, cefixime, cefotaxime, ceftazidime, ceftriaxone, cefuroxime, cephalosporins, cetirizine, chloramphenicol, chlordiazepoxide, chlorhexidine, chlorpheniramine, chlortalidone, choline, cyclosporin, cilastatin, cimetidine, ciprofloxacin, cisapride, cisplatin, clarithromycin, clavulanic acid, clomipramine, clonazepam, clonidine, clotrimazole, codeine, cholestyramine, cromoglycic acid, cyanocobalamin, cyproterone, desogestrel, dexamethasone, dexpanthenol, dextromethorphan, dextropropoxiphen, diazepam, diclofenac, digoxin, dihydrocodeine, dihydroergotamine, dihydroergotoxin, diltiazem, diphenhydramine, dipyridamole, dipyrone, disopyramide, domperidone, dopamine, doxycycline, enalapril, ephedrine, epinephrine, ergocalciferol, ergotamine, erythromycin, estradiol, ethinylestradiol, etoposide, Eucalyptus globulus, famotidine, felodipine, fenofibrate, fenoterol, fentanyl, flavine mononucleotide, fluconazole, flunarizine, fluorouracil, fluoxetine, flurbiprofen, furosemide, gallopamil, gemfibrozil, Ginkgo biloba, glibenclamide, glipizide, clozapine, Glycyrrhiza glabra, griseofulvin, guaifenesin, haloperidol, heparin, hyaluronic acid, hydrochlorothiazide, hydrocodone, hydrocortisone, hydromorphone, ipratropium hydroxide, ibuprofen, imipenem, indomethacin, iohexol, iopamidol, isosorbide dinitrate, isosorbide mononitrate, isotretinoin, ketotifen, ketoconazole, ketoprofen, ketorolac, labetalol, lactulose, lecithin, levocarnitine, levodopa, levoglutamide, levonorgestrel, levothyroxine, lidocaine, lipase, imipramine, lisinopril, loperamide, lorazepam, lovastatin, medroxyprogesterone, menthol, methotrexate, methyldopa, methylphenidate, methylprednisolone, metoclopramide, metoprolol, miconazole, midazolam, minocycline, minoxidil, misoprostol, morphine, N-methylephedrine, naftidrofuryl, naproxen, neomycin, nicardipine, nicergoline, nicotinamide, nicotine, nicotinic acid, nifedipine, nimodipine, nitrazepam, nitrendipine, nizatidine, norethisterone, norfloxacin, norgestrel, nortriptyline, nystatin, ofloxacin, omeprazole, ondansetron, pancreatin, panthenol, pantothenic acid, paracetamol, paroxetine, penicillins, phenobarbital, pentoxifylline, phenoxymethylpenicillin, phenylephrine, phenylpropanolamine, phenytoin, physostigmine, piroxicam, polymyxin B, povidone iodine, pravastatin, prazepam, prazosin, prednisolone, prednisone, bromocriptine, propafenone, propranolol, proxyphylline, pseudoephedrine, pyridoxine, quinidine, ramipril, ranitidine, reserpine, retinol, riboflavin, rifampicin, rutoside, saccharin, salbutamol, salcatonin, salicylic acid, simvastatin, somatotropin, sotalol, spironolactone, sucralfate, sulbactam, sulfamethoxazole, sulfasalazine, sulpiride, tamoxifen, tegafur, teprenone, terazosin, terbutaline, terfenadine, tetracaine, tetracycline, theophylline, thiamine, ticlopidine, timolol, tranexamic acid, tretinoin, triamcinolone acetonide, triamterene, triazolam, trimethoprim, troxerutin, uracil, valproic acid, verapamil, folinic acid, zidovudine, zopiclone, enantiomers thereof, organic and inorganic salts thereof, hydrates thereof, and mixtures thereof, in particular mixtures in synergistic proportions.

Other bio-agents suitable for time-controlled delivery according to the principles of the present invention include, but are not limited to, vitamins, especially those of the A group, of the B group (which means, besides B1, B2, B6 and B12, also compounds with vitamin B properties such as adenine, choline, pantothenic acid, biotin, adenylic acid, folic acid, orotic acid, pangamic acid, carnitine, p-aminobenzoic acid, myo-inositol and lipoic acid, as well as fully synthetic derivatives thereof such as disclosed for instance in U.S. Patent No. 6,667,298, U.S. Patent No. 6,506,912, U.S. Patent No. 6,482,812, U.S. Patent No. 6,316,642, U.S. Patent No. 6,127,559, U.S. Patent No. 6,017,907, U.S. Patent No. 5,945,410, U.S. Patent No. 5,936,133, U.S. Patent No. 5,936,105, U.S. Patent No. 5,830,885, U.S. Patent No. 5,536,713, U.S. Patent No. 5,403,832, U.S. Patent No. 5,389,622, U.S. Patent No. 5,281,731, U.S. Patent No. 5,274,142 and U.S. Patent No. 4,666,634), vitamin C, vitamins of the D group, E group, F group, H group, I and J groups, K group and P group.

This invention is also suitable for the time-controlled delivery of therapeutic agents (drugs) having very low solubility, e.g. a water-solubility as low as about 0.2 µg/ml. Non-limiting examples of such drugs include for instance hydrochlorothiazide, nimodipine, flufenamic acid, mefenamic acid, bendroflumethiazide, benzthiazide, ethacrinic acid, nitrendipine and diaminopyrimidines. Suitable examples of such poorly soluble diaminopyrimidines include, without limitation, 2,4-diamino-5-(3,4,5-trimethoxybenzyl) pyrimidine (trimethoprim), 2,4-diamino-5-(3,4-dimethoxy-benzyl) pyrimidine (diaveridine), 2,4 diamino-5-(3,4,6-trimethoxybenzyl) pyrimidine, 2,4-diamino-5-(2-methyl-4,5-dimethoxybenzyl) pyrimidine (ormeto-prim), 2,4-diamino-5-(3,4-dimethoxy-5-bromobenzyl) pyrimidine, 2,4-diamino-5-(4-chloro-phenyl)-6-ethylpyrimidine (pyrimethamine), and analogues thereof.

This invention is suitable for the time-controlled delivery of therapeutic agents (drugs) which (according to well established pharmacological principles) are further formulated with one or more pharmaceutically acceptable excipients, such as emulsifiers or surface-active agents, thickening agents, gelling agents, disintegrating agents, sweeteners or other additives such as described hereinafter.

Emulsifiers or surface-active agents suitable for bio-agent (including therapeutic agents) formulations for use in the present invention include, but are not limited to, water-soluble natural soaps and water-soluble synthetic surface-active agents. Suitable soaps include alkaline or alkaline-earth metal salts, unsubstituted or substituted ammonium salts of higher, preferably saturated, fatty acids (C₁₀-C₂₂), e.g. the sodium or potassium salts of oleic or stearic acid, or of natural fatty acid mixtures obtainable form coconut oil, palm oil or tallow oil. Synthetic surface-active agents (surfactants) include anionic, cationic and nonionic surfactants, e.g. sodium or calcium salts of polyacrylic acid; sulphonated benzimidazole derivatives preferably containing 8 to 22 carbon atoms; alkylarylsulphonates; and fatty sulphonates or sulphates, usually in the form of alkaline or alkaline-earth metal salts, unsubstituted ammonium salts or ammonium salts substituted with an alkyl or acyl radical having from 8 to 22 carbon atoms, e.g. the sodium or calcium salt of lignosulphonic acid or dodecylsulphonic acid or a mixture of fatty alcohol sulphates obtained from natural fatty acids, alkaline or alkaline-earth metal salts of sulphuric or sulphonic acid esters (such as sodium lauryl sulphate) and sulphonic acids of fatty alcohol/ethylene oxide adducts. Examples of alkylarylsulphonates are the sodium, calcium or alcanolamine salts of dodecylbenzene sulphonic acid or dibutyl-naphtalenesulphonic acid or a naphtalene-sulphonic acid/formaldehyde condensation product. Also suitable are the corresponding phosphates, e.g. salts of phosphoric acid ester and an adduct of p-nonylphenol with ethylene and/or propylene oxide) and the like.

Suitable emulsifiers further include, but are not limited to, partial esters of fatty acids (e.g. lauric, palmitic, stearic or oleic) or hexitol anhydrides (e.g., hexitans and hexides) derived from sorbitol, such as commercially available polysorbates. Other emulsifiers which may be used include, but are not limited to, adducts of polyoxyethylene chains (1 to 40 moles ethylene oxide) with non-esterified hydroxyl groups of the above partial esters, such as the surfactant commercially available under the trade name Tween 60 from ICI Americas Inc.; and the poly(oxyethylene)/poly(oxypropylene) materials marketed by BASF under the trade name Pluronic.

Suitable structure-forming, thickening or gel-forming agents for the bio-agents for use in the present invention include, but are not limited to, highly dispersed silicic acid, such as the product commercially available under the trade name Aerosil; bentonites; tetraalkyl ammonium salts of montmorillonites (e.g. products commercially available under the trade name Bentone) wherein each of the alkyl groups may contain from 1 to 20 carbon atoms; cetostearyl alcohol and modified castor oil products (e.g. a product commercially available under the trade name Antisettle).

Gelling agents which may be included into the bio-agent formulation of the present invention include, but are not limited to, cellulose derivatives such as carboxymethylcellulose, cellulose acetate and the like; natural gums such as arabic gum, xanthum gum, tragacanth gum, guar gum and the like; gelatin; silicium dioxide; synthetic polymers such as carbomers, and mixtures thereof. Gelatin and modified celluloses represent a preferred class of gelling agents.

Other optional excipients which may be present in the bio-agent formulation of the present invention include, but are not limited to, additives such as magnesium oxide; azo dyes; organic and inorganic pigments such as titanium dioxide; UV-absorbers; stabilisers; odor masking agents; viscosity enhancers; antioxidants such as, for example, ascorbyl palmitate, sodium bisulfite, sodium metabisulfite and the like, and mixtures thereof; preservatives such as, for example, potassium sorbate, sodium benzoate, sorbic acid, propyl gallate, benzylalcohol, methyl paraben, propyl paraben and the like; sequestering agents such as ethylene-diamine tetraacetic acid; flavoring agents such as natural vanillin; buffers such as citric acid or acetic acid; extenders or bulking agents such as silicates, diatomaceous earth, magnesium oxide or aluminum oxide; densification agents such as magnesium salts; and mixtures thereof.

Time-controlled explosion bio-agent release systems and pulsed bio-agent delivery systems according to this invention may take different forms in terms of shape, size, composition and number of layers. For instance, the pulsed delivery system may be in the form of beads or granules of various shapes, sizes and size distribution.

The present invention is not limited to releasing the one or more bio-agents as a single pulse. In specific cases, it may be beneficial to provide multiple pulsed delivery or multiple explosion release of the same bio-agent or two or more different bio-agents. This may be effected by providing the delivery system, e.g. the biodegradable core of said delivery system, with a mixture of at least two swelling agents having different degradation rates such as to provide two or more different lag times for the one or more bio-agents. In a specific embodiment for this purpose, the degradable core of the delivery system may comprise at least (i) a first population of microcapsules including a first bio-agent and a first swelling agent and (ii) a second population of microcapsules including a second bio-agent and a second swelling agent, so that the first bio-agent is released or delivered after a first lag time and the second bio-agent is released or delivered after a second lag time, the said second lag time being substantially different from the said first lag time. In this embodiment, the first bio-agent may be different from or the same as the second bio-agent, thus providing additional flexibility for the biological, e.g. therapeutic or prophylactic, treatment. As previously mentioned, each of the first and subsequent lag times may independently vary within very broad ranges from about 1 hour to about 24 hours.

The pulsed delivery or explosion release systems of the present invention, as well as the drug-loaded microcapsules obtained by loading hollow microcapsules, are suitable for a number of different ways of administration of therapeutic agents to mammals (including human beings) such as, but not limited to, oral administration, parenteral administration, subcutaneous administration, topical administration, vaccination and the like, depending upon the type of therapeutic agent, the desired release profile, the desired locus of action, and upon the condition of the mammal to be treated.

In another aspect, the invention relates to a method of protecting plants or crops by releasing one or more bio-agents selected from the group consisting of fertilisers, anti-microbial agents, insecticides, fungicides, herbicides and pesticides onto said plants or crops, wherein said one or more bio-agents is (are) included in a time-controlled explosion bio-agent release system or pulsed bio-agent delivery system comprising at least (i) an outer semi-permeable membrane wherein bio-agent release or delivery is caused by explosion of said semi-permeable membrane and begins after a lag time and at least (ii) a core comprising a bio-agent and a swelling agent, wherein said swelling agent is a degradable biocompatible oligomer or polymer aqueous solution or hydrogel wherein degradation occurs by cleavage of the polymer backbone and/or, in the case of a hydrogel, by cleavage of cross-linking bonds within said hydrogel, characterised in that said outer semi-permeable membrane (i) consists of two or more alternating layers of at least one positively charged biocompatible polypeptide selected from the group consisting of poly-L-arginine and poly-L-ornithine and at least one biocompatible polyanion selected from the group consisting of chondroitin sulfate, poly-L-glutamic acid and poly-L-aspartic acid.

Said method of protecting plants or crops preferably comprises spraying said time-controlled explosion bio-agent release system or pulsed bio-agent delivery system onto the plants or crops to be protected, either in solid form or, even more preferably for dose control and reproducibility, as a dispersion in a suitable liquid medium such as, but not limited to, water.

The following set of examples provides a selection of a few appropriate working embodiments for this invention which should be understood as purely illustrative and without any limiting intention, as well as comparative data for a better understanding of the unexpected advantage of this invention.

### EXAMPLE 1 (comparative) ― preparation of Dex-HEMA ―DMAEMA hydrogels

Biodegradable dextran-based positively charged microgels with an average diameter of 7 µm were prepared by co-polymerizing dextranhydroxyethyl methacrylate (Dex-HEMA) with dimethylaminoethyl methacrylate (DMAEMA). In detail, Dex-HEMA -DMAEMA microgels with a water content of 70 % by weight were made as follows. Aqueous solutions of Dex-HEMA (25 % by weight, for instance produced according to the general procedure described by O. Franssen et al. in Int. J. Pharm. (1998) 168:1), polyethylene glycol with a number average molecular weight of 20,000 (24 % by weight) and DMAEMA (25 mM) were vigorously mixed in a 1:20 volume ratio to obtain a water-in-water emulsion. After 10 minutes, 100 µl N,N,N',N'-tetramethylenediamine (TEMED), pH neutrelized with 4 N HCl, and 9 mg potassium peroxide (KPS) were added in order to cross-link Dex-HEMA. Washing and centrifugation steps with 50 mL Milli-Q water was performed three times in order to remove residual KPS and TEMED. The starting materials used in this procedure were:
- dextran (commercially available from Fluka, obtained from *Leuconostoc ssp.)* with an average number molecular weight of 19,000,
- TEMED and DMAEMA commercially available from Aldrich, and
- KPS commercially available from Merck.

The degree of substitution (hereinafter DS) of the Dex-HEMA used in this example was determined by proton nuclear magnetic resonance spectroscopy (H-NMR) in D₂O with a Gemini 300 spectrometer (Varian) and was found to be 2.5.

### EXAMPLE 2 (comparative) ― layer-by-layer (LbL) coating of Dex-HEMA ― DMAEMA hydrogels

Dex-HEMA-DMAEMA microgels obtained in example 1 were coated by the consecutive adsorption of oppositely charged polyelectrolytes using the following centrifugation technique. Sodium polystyrenesulfonate (PSS) served as polyanion while polyallylamine hydrochloride (PAH) served as a polycation. In detail, 500 µl (15 mg/ml) of the microgels of example 1 were dispersed in 1 mL of a polyelectrolyte solution (2 mg/mL in 0.5M NaCl, and shaken to allow adsorption for 15 minutes. The excess polyelectrolyte was then removed by two successive centrifugation steps. This procedure however faced some microgel agglomeration problems.

This adsorption procedure was repeated until 3 polyelectrolyte layers were obtained and was monitored by measuring the electrophoretic mobility after each adsorption step. The initial ζ-potential of the microgels was + 29 mV and oscillated steadily between― 50 mV and + 50 mV upon alternating adsorption of PSS and PAH. Rhodamine-labeled PAH was also used to visualize the coating, and confocal scanning laser microscopy (CSLM) images (using a MRC1024 Bio-Rad scanning system equipped with a 60x water immersion objective) of the coated microgels were made, wherein a clear fluorescent ring was observed, thus indicating the formation of a polyelectrolyte membrane surrounding the microgels.

### EXAMPLE 3 (comparative) ― pH-dependent permeability of the membrane of layer-by-layer (LbL) coated dex-HEMA-DMAEMA microgels

The permeability of the (PSS/PAH) coating of the layer-by-layer coated microgels obtained in example 2 to template bio-active molecules of different molecular weights and in different pH conditions was investigated, using the same CSLM equipment as in example 2. In detail, permeability experiments were performed in 96 well plates by mixing 50 µl of a fluorescein isothiocyanatedextran (FITC-D) solution (1 mg/ml) with 50 µl of a microgel dispersion, both in a 0.1 M carbonate buffer at pH 9 and in a 0.1 L phosphate buffer at pH 7. The template bio-active molecules used for these experiments were FITC-D with average number molecular weights 4,000 and 20,000 respectively, commercially available from Aldrich. Experiments of fluorescence recovery after photobleaching (FRAP) were performed by bleaching a circular region inside the capsules. Fluorescence recovery was quantified by calculating the ratio of the fluorescence intensity of the recovering region to the fluorescence intensity of the non-bleached region in the same capsule.

These experiments have shown that the permeability of the (PSS/PAH) coating of the layer-by-layer coated microgels obtained in example 2 was pH dependent:
- at pH 7 the coated microgels were permeable to both FITC-D with an average number molecular weight of 4,000 and FITC-D with an average number molecular weight of 20,000,
- while at pH 9 the coated microgels were impermeable to both FITC-D with an average number molecular weight of 4,000 and FITC-D with an average number molecular weight of 20,000.

### EXAMPLE 4 (comparative) ― behaviour of layer-by-layer (LbL) coated dex-HEMA-DMAEMA microgels during degradation

The behaviour of the (PSS/PAH) coating of the layer-by-layer coated microgels obtained in example 2 was investigated as follows. The coated microgels were incubated at 37 °C at pH 7 as well as at pH 9.

CLSM images of the coated microgels after 5 days of incubation at pH 7 were taken. The occurrence of microgel degradation at pH 7 was further confirmed by studying the mobility of encapsulated FITC-D with an average number molecular weight of 150,000 (commercially available from Aldrich) by FRAP before and after incubating the coated microgels for 10 days at pH 7. This has clearly shown that fluorescence significantly recovers, indicating that the FITC-D chains became mobile due to degradation of the network in the microgels. Nevertheless the microgels were degraded, and clearly explosion of the capsules did not occur.

CLSM images of the coated microgels after 1 day of incubation at pH 9 were taken and have clearly shown that only remnants of broken polyelectrolyte shells were visible after degradation of the microgels. In order to confirm self-explosion of the capsules, the following experiment was performed. The coated microgels obtained in example 2 with encapsulated FITC-D with an average number molecular weight of 150,000 were placed at 80 °C for 4 minutes in a 0.1 M carbonate buffer at pH 9. The capsules were subsequently put under the CLSM equipment at 40 °C and followed over time. Snapshots of the capsules taken after respectively 60 minutes, 75 minutes, 90 minutes and 105 minutes clearly show rupturing of the coating and sudden release of the encapsulated FITC-D.

### EXAMPLE 5 - preparation of polypeptide-coated Dex-HEMA-DMAEMA hydrogel self-exploding microcapsules

The polypeptides poly-L-arginine (ARG_{low} with a number average molecular weight ranging from 15,000 to 70,000; ARG_{high} with a number average molecular weight ranging from 70,000 to 200,000), poly-L-glutamic acid (GLU_{low} with a number average molecular weight ranging from 15,000 to 50,000; GLU_{high} with a number average molecular weight ranging from 50,000 to 100,000) and poly-L-aspartic acid (ASP with a number average molecular weight ranging from 15,000 to 50,000) used in this preparation were purchased from Sigma-Aldrich.

The positively charged Dex-HEMA-DMAEMA spherically shaped microgels with an average diameter of 10 µm made according to the procedure of example 1 were coated with combinations of a polycation and a polyanion selected from the above group of polypeptides. The polyelectrolyte coating was deposited by alternating immersion of the microgels in solutions of 1 mg/ml of the relevant polyelectrolyte containing 0.5 M NaCl, followed by two washing steps, by analogy to the method described in example 2. Opposite to the manufacturing procedure of example 2, this procedure did not result in any microgel agglomeration problem.

The stepwise adsorption of oppositely charged polyelectrolytes was monitored by measuring the electrophoretic mobility of the particles. The initial ζ-potential of the dex-HEMA-DMAEMA microgels was + 30 mV and changed alternately between +50 and ―50 mV upon adsorption of oppositely charged polyelectrolytes.

Different polypeptide combinations were used in this study and, depending upon their molecular weight, different structures were observed by confocal imaging (CSLM) of the obtained microcapsules, the polyelectrolyte coating being fluorescent red-labelled by using rhodamine-labelled ARG.

The following combinations were tried, the subscript indicating the number of layers: (GLU_{high}/ARG_{high})₄, (GLU_{low}/ARG_{low})₄, (GLU_{high},/ARG_{low})₄, (GLU_{low},/ARG_{high})₄, (ASP/ARG_{high})₄ and (ASP/ARG_{low})₄. These combinations were tested on their ability to explode after addition of sodium hydroxide for accelerating the degradation of the microgels. These experiments have shown that it was necessary to use poly-L-arginine, preferably high molecular weight poly-L-arginine, as a positively charged polypeptide in order to obtain self-exploding microcapsules. There is a tendency to decrease the proportion of self-exploding microcapsules when the number average molecular weight of poly-L-arginine decreases, presumably because it leads to a polyelectrolyte shell which is too permeable, thus leading to outwards diffusion instead of osmotic pressure build-up.

The above experiment was repeated while incorporating FITC-D with an average number molecular weight of 150,000 as a model bio-agent inside the microgels. Incubating the microcapsules in a solution containing 0.25 M sodium hydroxide resulted, when poly-L-aspartic acid was used in combination with poly-L-arginine, into the microcapsules starting to swell and explode, thus resulting into the release of the encapsulated FITC-D. In a further experiment, FITC-D was replaced with a low molecular weight model bio-agent, 6-carboxyfluorescein with a molecular weight of 367, and resulted in similar observations.

### EXAMPLE 6 - use of polypeptide combinations for preparing hollow capsules

The positively charged Dex-HEMA-DMAEMA spherically shaped microgels with an average diameter of 10 µm made according to the procedure of example 1 were coated with 4 bilayers of dextran sulfate / poly-L-arginine combinations using the same electrostatic and centrifugation procedure as in example 5. In a second step the microgel core was dissolved by incubating the LbL-coated microgels in a 0.1 M sodium hydroxide (NaOH) solution for 30 minutes. This alkaline environment causes the carbonates esters, which connect the polymerized methacrylate groups with the dextran chains, to hydrolyze leading to the formation of oligomethacrylates and the original dextran chains, used for the synthesis of dex-HEMA, as degradation products. To remove the degradation products and the excess NaOH, the capsules were washed several times with pure water, thus resulting in hollow microcapsules that were observed by confocal imaging, using the same CLSM equipment as in example 2.

Fluorescent labelling was also achieved by incorporation of FITC-D with an average number molecular weight of 150,000 as a model bio-agent inside the hydrogel microcapsules during their synthesis and by using RBITC-labelled poly-L-arginine. From the confocal images it was observed that no fluorescence remains after degradation of the microgels, indicating the outwards diffusion of FITC-D upon dissolution of the microgel core.

### EXAMPLE 7 ― method for drug loading hollow capsules prepared from polypeptide combinations

For applications in the field of drug delivery, it is necessary to provide a method to obtain filled microcapsules. Therefore the hollow capsules prepared according to example 6 were incubated for 30 minutes in a solution containing 1 mg/ml fluorescein isothiocyanate-bovine serum albumin (FITC-BSA) as a model bio-agent. Confocal images obtained by using the same CLSM equipment as in example 2 have shown the unexpected result of FITC-BSA accumulated inside the microcapsules leaving the background to remain dark. Even when several washing steps are performed to remove eventually non-adsorbed FITC-BSA, the microcapsules retained their green fluorescence, thus indicating a permanent encapsulation of the model bio-agent.

## Claims

1. A microcapsule of a degradable biocompatible hydrogel being coated by means of two or more alternating layers of at least one positively charged biocompatible polypeptide selected from the group consisting of poly-L-arginine and poly-L-ornithine and at least one biocompatible polyanion selected from the group consisting of dextran sulfate, chondroitin sulfate, poly-L-glutamic acid and poly-L-aspartic acid.

2. A microcapsule according to claim 1, wherein said degradable biocompatible hydrogel is a modified dextran.

3. A microcapsule according to claim 2, wherein dextran is modified by means of at least one hydroxy-C₁₋₈ alkyl acrylate or methacrylate.

4. A microcapsule according to any of claims 1 to 3, having a size from about 2 µm to about 20 µm.

5. A microcapsule according to any of claims 1 to 4, wherein the number of alternating layers is from 3 to 10.

6. A microcapsule according to any of claims 1 to 5, wherein the molecular weight of the biocompatible polyanion is within a range from about 5,000 to about 100,000.

7. A microcapsule according to any of claims 1 to 6, wherein the number average molecular weight of the positively charged biocompatible polypeptide is within a range from about 15,000 to about 200,000.

8. A microcapsule according to any of claims 1 to 7, wherein degradable biocompatible hydrogel is loaded with one or more bio-agents.

9. A microcapsule according to claim 8, wherein the weight proportion of said one or more bio-agents within said degradable biocompatible hydrogel is from 1% to 10%.

10. A microcapsule according to any of claims 1 to 9, being able to explode after incubation at 37°C at a pH below 7.5.

11. A microcapsule according to claim 10, wherein said at least one positively charged biocompatible polypeptide is poly-L-arginine, and wherein said at least one negatively charged biocompatible polyanion is selected from the group consisting of chondroitin sulfate, poly-L-glutamic acid with a molecular weight below 50,000, and poly-L-aspartic acid.

12. A microcapsule according to any of claims 1 to 9, being a hollow capsule.

13. A microcapsule according to claim 12, wherein said at least one positively charged biocompatible polypeptide is poly-L-arginine, and wherein said at least one negatively charged biocompatible polyanion is dextran sulfate.

14. A microcapsule according to claim 8 or claim 9, wherein said bio-agent is selected from the group consisting of therapeutic drugs and synthetic molecules, proteins, nucleic acids, vitamins, hormones, nutrients, aromas, fertilisers, anti-microbial agents, insecticides, fungicides, herbicides and pesticides.
